(19) Europäisches Patentamt
European Patent Office
Office européen des brevets

(11) Veröffentlichungsnummer : **0 001 275**
**B2**

(12) # NEUE EUROPÄISCHE PATENTSCHRIFT

(45) Veröffentlichungstag der neuen Patentschrift : **10.09.86**

(51) Int. Cl.[4] : **C 07 C143/70**, C 07 C139/02

(21) Anmeldenummer : **78100916.2**

(22) Anmeldetag : **18.09.78**

(54) **Verfahren zur Herstellung von Sulfonsäurechloriden.**

(30) Priorität : **28.09.77 DE 2743540**

(43) Veröffentlichungstag der Anmeldung : **04.04.79 Patentblatt 79/07**

(45) Bekanntmachung des Hinweises auf die Patenterteilung : **08.04.81 Patentblatt 81/14**

(45) Bekanntmachung des Hinweises auf die Entscheidung über den Einspruch : **10.09.86 Patentblatt 86/37**

(84) Benannte Vertragsstaaten :
**BE CH DE FR GB LU NL**

(56) Entgegenhaltungen :
**DE-A- 2 135 087**
**DE-A- 2 414 498**
**DE-A- 2 635 279**
**DE-A- 2 635 281**
**DE-C- 752 572**
**DE-C- 1 593 878**
**US-A- 2 704 295**
**US-A- 3 772 373**
**HOUBEN-WEYL "Methoden der organischen Chemie" 4. Auflage, Band IX, 1955, Georg Thieme Verlag, Stuttgart, Seiten 568-569**
**ULLMANNS ENCYKLOPAEDIE DER TECHNISCHEN CHEMIE 4. Auflage, Band 8, 1974, Verlag Chemie, Weinheim, Seite 420**
**Nikolai N. Woroshzov, "Grundlagen der Synthese von Zwischenprodukten und Farbstoffen", Akademie-Verlag, Berlin (1966), Seite 75, Abschnitt 2.3.7., und Seite 76**
**Houben-Weyl, Methoden der organischen Chemie, 4. Auflage, Band IX (1955), Seiten 510 bis 514**
**"Organikum", 4. Auflage, VEB Deutscher Verlag der Wissenschaften, Berlin 1964, Seite 515**
**"Beitrag zur Kenntinis der Sulfonierung aromatischer Verbindungen mit Schwefeltrioxid", Dissertation von R. Fleury (1966)**
**"Sulfonation and Related Reactions" von E.E. Gilbert (1965), Seiten 66 bis 70**

(73) Patentinhaber : **BAYER AG**
**Konzernverwaltung RP Patentabteilung**
**D-5090 Leverkusen 1 Bayerwerk (DE)**

(72) Erfinder : **Schenk, Wolfgang, Dr.**
**Odenthaler Strasse 62/64**
**D-5090 Leverkusen 1 (DE)**
Erfinder : **Blank, Heinz Ulrich, Dr.**
**Am Geusfelde 35**
**D-5068 Odenthal (DE)**
Erfinder : **Hagedorn, Ferdinand, Dr.**
**Domblick 16**
**D-5090 Leverkusen 31 (DE)**
Erfinder : **Evertz, Werner**
**Wolffstrasse 1**
**D-4019 Monheim (DE)**

**Chim. Chim. Technol. 14 (19), Seiten 1530 bis 1532, (1971) deutsche Übersetzung**
**Organikum, 8. Aufl., Seite 538 (1968)**
**Ullmann, 4. Aufl., Band 8, Seite 420 (1974)**
**J. Org. Chem. 20 (1955), Seiten 455 bis 465**
**J. Of General Chemistry of the USSR, Vol. XXIII (1953), Seiten 103-110**
**Houben-Weyl, 4. Aufl., Band IX, Seite 552**
**BIOS 986, I, 56**
**Die Akte enthält technische Angaben, die nach dem Eingang der Anmeldung eingereicht wurden und die nicht in dieser Patentschrift enthalten sind.**



Jouve, 18, rue St-Denis, 75001 Paris, France

**Beschreibung**

Die Erfindung betrifft ein Verfahren zur Herstellung von Sulfonsäurechloriden durch Umsetzung von aromatischen Verbindungen mit Chlorsulfonsäure und Thionylchlorid.

Es ist bekannt (Ullmanns Enzyklopädie der technischen Chemie, 4. Aufl., Band 8 (1974), Seite 420). Benzolsulfonsäurechlorid durch Umsetzung von Benzol mit überschüssiger Chlorsulfonsäure herzustellen. Die Verwendung von überschüssiger Chlorsulfonsäure ist besonders im Hinblick auf den Umweltschutz ein erheblicher Nachteil dieses Verfahrens, da die überschüssige Chlorsulfonsäure bei der Aufarbeitung des Reaktionsgemisches mit Wasser zu Chlorwasserstoff und Schwefelsäure hydrolisiert wird und zusammen mit der zusätzlich auch als Nebenprodukt entstehenden Schwefelsäure, sowie zusammen mit erheblichen Mengen nicht zum Sulfonsäurechlorid umgesetzter Benzolsulfonsäure als sogenannte Dünnsäure erhalten wird. Die Aufarbeitung und Beseitigung dieses Zwangsanfalls an Dünnsäure führt zu erheblichen Aufwendungen. Bei einfacher Neutralisation der Dünnsäure ergibt sich ein entsprechender Salzgehalt des Abwassers, der ebenfalls aus Gründen des Umweltschutzes unerwünscht ist und eine aufwendige Entsalzung des Abwassers notwendig macht.

Es wurde ein Verfahren zur Herstellung von Sulfonsäurechloriden der Formel

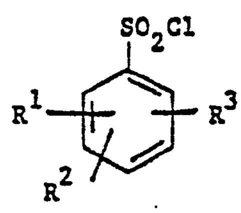

(I)

worin $R^1$, $R^2$ und $R^3$ gleich oder verschieden sind und Wasserstoff, einen niederen Alkyl- oder einen Cycloalkylrest, Halogen, Aryl, Aralkyl, Aryläther oder einen Rest $-SO_2Cl$, $-SO_2$Aryl,

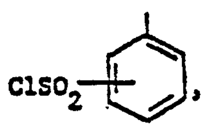, 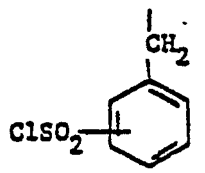, 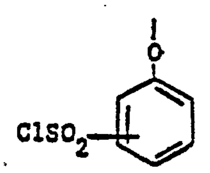 oder 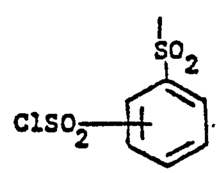

bedeuten

oder wo benachbarte Reste $R^1$ und $R^2$ zu einem gegebenenfalls durch eine Sulfonsäurechloridgruppe substituierten cycloaliphatischen aromatischen oder heteroaromatischen Ring verknüpft sind, wobei die Reste $R^1$ bis $R^3$ durch weitere Reste wie Halogen, Niederalkyl, Aryl, Aroxy, Alkoxy und Aralkyl substituiert sein können, durch Umsetzung einer aromatischen Verbindung der Formel

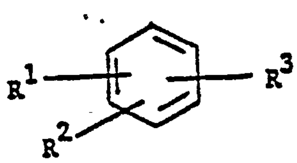

(II)

worin $R^1$, $R^2$ und $R^3$ die oben genannte Bedeutung haben, mit Chlorsulfonsäure und Thionylchlorid gefunden, bei dem man zuerst die aromatische Verbindung und Chlorsulfonsäure in stöchiometrischer Menge bis zu einem Überschuß von 20 Mol-%, bezogen auf jede einzuführende Sulfonsäure-chlorid-Gruppe, in Gegenwart eines Sulfonierungshilfsmittels und gegebenenfalls in Gegenwart eines Lösungsmittels, zur Reaktion bringt und dann mit überschüssigem Thionylchlorid umsetzt.

Niedere Alkylreste ($R^1$ bis $R^3$) können geradkettige oder verzweigte Alkylreste mit 1 bis 6, bevorzugt 1 bis 4, Kohlenstoffatome sein. Beispielsweise seien genannt: Methyl, Äthyl, Propyl, Isopropyl, Butyl, Isobutyl, Pentyl, Isopentyl, Hexyl und Isohexyl.

Als Cycloalkylreste ($R^1$ bis $R^3$) seien beispielswiese Cyclopentyl und Cyclohexyl, bevorzugt Cyclohexyl, genannt.

Als Halogene ($R^1$ bis $R^3$) seien Fluor, Chlor, Brom und Jod, bevorzugt Fluor, Chlor und Brom, genannt.

Als Arylrest ($R^1$ bis $R^3$) sei beispielsweise Phenyl genannt.

Als Aralkylreste ($R^1$ bis $R^3$) kommen beispielsweise solche mit 6 bis 18 Kohlenstoffatomen, deren aliphatischer Teil 1 bis 6 Kohlenstoffatome enthält und deren aromatischer Teil einen Rest aus der Benzolreihe darstellt, infrage. Beispielsweise seien die folgenden araliphatischen Reste genannt: Benzyl, ß-Äthyl-phenyl, γ-Propyl-phenyl, und ß-Phenyl-n-hexyl, bevorzugt Benzyl.

Als Arylätherrest ($R^1$ bis $R^3$) sei insbesondere der Phenoxyrest genannt.

Durch die Verknüpfung der benachbarten Reste $R^1$ und $R^2$ zu einem cycloaliphatischen oder aromatischen Ring entstehen kondensierte Ringsysteme, wie Indan, Tetralin und Naphthalin, bevorzugt Naphthalin.

Es ist selbstverständlich möglich, daß die Reste $R^1$ bis $R^3$ durch weitere Reste substituiert sind. Beispielsweise seien Halogen, Niederalkyl, Aryl, Aroxy, Alkoxy und Aralkyl genannt.

Als bevozugte aromatische Verbindungen seien Verbindungen der Formel

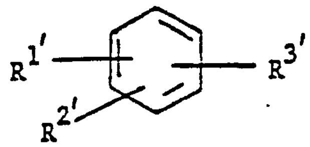

(III)

worin

$R^{1'}$, $R^{2'}$ und $R^{3'}$ gleich oder verschieden sind und Wasserstoff, einen Alkylrest mit 1 bis 4 Kohlenstoffatomen, Fluor, Chlor, Brom, Phenyl, $-SO_2Cl$, $-SO_2$Phenyl, Phenoxy oder Benzyl bedeuten oder wo benachbarte Reste

$R^{1'}$ und $R^{2'}$ zu einem cycloaliphatischen, aromatischen oder heteroaromatischen Ring mit 5 oder 6 Ringgliedern verknüpft sind, genannt.

Beispielsweise seien die folgenden aromatischen Verbindungen genannt :

Benzol, Toluol, Äthylbenzol, Isopropylbenzol (Cumol), Tetralin, o-Xylol, m-Xylol, p-Xylol, Diphenyl, Diphenylmethan, Chlorbenzol, 1-Chlornaphthalin, 2-Chlornaphthalin, o-Chlortoluol, 1,2-, 1,3- und 1,4-Dichlorbenzol, 2,3-, 2,4-, 2,5-, 3,4- und 2,6-Dichlor-touol, 2,3-, 2,4-, 2,6-, 3,4- und 2,5-Dimethylchlorbenzol, Brombenzol, Fluorbenzol, 1,2,3- und 1,2,4-Trichlorbenzol, Diphenyläther, Naphthalin, 1- und 2-Methylnaphthalin und 2-, 3- und 4-Bromtoluol, Diphenylsulfon, Benzolsulfochlorid, Toluolsulfochlorid, Diphenylensulfon, Diphenylenoxyd. Insbesondere wird die Umsetzung von Benzol bevorzugt.

Das erfindungsgemäße wird Verfahren in Gegenwart eines Sulfonierungshilfsmittels durchgeführt. Sulfonierungshilfsmittel nach dem erfindungsgemäßen Verfahren sind im allgemeinen Verbindungen, die gegenüber Chlorsulfonsäure als Lewis-Basen wirksam werden können.

Als Sulfonierungshilfsmittel seien beispielsweise genannt :

1. Carbonsäuren und ihre Derivate (z. B. Essigsäure, Propionsäure, Buttersäure, Pivalinsäure, Valeriansäure, Capronsäure, Adipinsäure, Azelainsäure, halogenierte Carbonsäuren wie Mono- Di- und Trichloressigsäure, Acetanhydrid, Propionsäureanhydrid, Buttersäureanhydrid, Bernsteinsäureanhydrid, Phthalsäureanhydrid, Acetylchlorid, Propionsäurechlorid, Buttersäurechlorid, Adipinsäurechlorid, Essigsauremethyl-äthyl, -butylester, propionsäureester, Buttersäureester, Formamid, Dimethylformamid, Phthalimid, Succinimid).

2. Harnstoff und Alkylharnstoffe,

3. Phosphorverbindungen (z. B. Phosphorsäure, org. Phosphorderivate wie Triäthyl-, Trimethyl-, Tributylphosphat, Triäthyl-, Trimethylphosphit),

4. Äther (z. B. Diäthyläther, Dioxan, Tetrahydrofuran),

5. Ketone (z. B. Aceton, Methyläthyketon, Methylpropylketon, Methylisobutylketon, Cyclopentanon, Cyclohexanon) und

6. Amine (z. B. primäre, sekundäre, tertiäre aliphatische Amine, z. B. Mono-, Di-, Triäthylamin, Isopropylamin, tert.-Butylamin, Cyclohexylamin, Morpholin, Äthanolamin, heterocyclische Amine wie Pyridin, Imidazol) bzw. deren Salze.

Besonders bevorzugt werden Carbonsäuren bzw. deren Derivate und Phosphorverbindungen, insbesondere bevorzugt hilfs werden Di-methylformamid, Essigsäure und Phosphorsäure.

Das Sulfonierungsmittel kann in einer Menge von 0,5 bis 10 Mol-%, vorzugsweise von 1 bis 8 Mol-% und insbesondere von 2 bis 5 Mol-% angewandt werden ; im allgemeinen kann es zu Beginn der Reaktion ganz oder teilweise mit vorgelegt werden. Der gegebenenfalls nicht eingesetzte Teil wird dann im weiteren Verlauf der ersten Reaktionsstufe mit zugetropft bzw. portionsweise zugegeben.

Nach dem erfindungsgemäßen Verfahren wird beispielsweise in der ersten Verfahrensstufe die Chlorsulfonsäure zusammen mit dem Sulfonierungshilfsmittel vorgelegt und die aromatische Verbindung nach Maßgabe der Reaktionsgeschwindigkeit, die anhand der Chlorwasserstoffentwicklung festgestellt werden kann, zugegeben.

Die Chlorsulfonsäure wird im allgemeinen in stöchiometrischen Mengen, bezogen auf die Zahl der einzuführenden Sulfonsäurechloridgruppen eingesetzt. Es kann jedoch vorteilhaft sein, einen geringen Überschuß bis zu 20 Mol-% und insbesondere bis zu 5 Mol-%, einzusetzen.

Die Reaktionsgemische aus der aromatischen Verbindung und Chlorsulfonsäure im angegebenen Verhältnis sind innerhalb des angeführten Temperaturbereichs von 30 bis 130 °C auch nach abgelaufener Umsetzung meistens flüssig und vor allem leicht rührbar, sodaß die Verwendung eines bezüglich Chlorsulfonsäure inerten organischen oder anorganischen Lösungsmittels zwar möglich, aber gewöhnlich nicht notwendigerweise erforderlich ist. Die Umsetzung mit dem Thionylchlorid kann daher gegebenenfalls ebenfalls ohne Lösungsmittelzusatz, direkt im Anschluß an den ersten Verfahrensschritt

durchgeführt werden, sodaß das erfindungsgemäße Verfahren als « Eintopfverfahren » durchgeführt werden kann.

Als gegebenenfalls eingesetzte Lösungs- oder Verdünnungsmittel kommen in Betracht : flüssiges Schwefeldioxid, Sulfonylchlorid, Kohlenwasserstoffe und Halogenkohlenwasserstoffe, insbesondere Alkane und Halogenalkane wie Chloroform, Tetrachlorkohlenstoff, Methylenchlorid, Di-, Tri- und Tetrachloräthylen, Di-, Tri-, Tetra- und Pentachloräthan, 1,1,2-Trichlor-1,2,2-trifluoräthan und Tetrafluoräthylen.

Im allgemeinen wird die erste Stufe des erfindungsgemäßen Verfahrens im Temperaturbereich von etwa 0 bis 150 °C, vorzugsweise von 10 bis 140 °C, insbesondere bevorzugt von 20 bis 100 °C, durchgeführt.

Ist die Reaktionstemperatur höher als bei Rückflußbedingungen bei Normaldruck, so arbeitet man zweckmäßig bei einem Druck, der dem Dampfdruck des Reaktionsgemischs bei den jeweils gewählten Bedingungen entspricht.

Bis etwa 100 °C kann auch drucklos durch Eindosieren der flüssigen aromatischen Verbindung bzw. durch Einleiten der gasförmigen aromatischen Verbindung gearbeitet werden, ohne daß größere Mengen der aromatischen Verbindungen über einen zweckmäßigerweise verwendeten Rückflußkühler entweichen.

Nach beendeter Umsetzung kann es zweckmäßig sein, gegebenenfalls nicht umgesetzte aromatische Verbindung beispielsweise durch Destillation aus der Reaktion zu entfernen.

In der zweiten Verfahrensstufe des erfindungsgemäßen Verfahrens wird das Thionylchlorid im Überschuß bezogen auf die eingesetzte aromatische Verbindung angewendet. Im allgemeinen kann man eine Menge von bis zu 5 Mol Thionylchlorid, vorzugsweise von 1,2 bis 3 Mol, insbesondere von 1,5 bis 2 Mol Thionylchlorid, bezogen auf jede einzuführende Sulfonsäurechlorid-Gruppe einsetzen.

Für das erfindungsgemäße Verfahren ist es nicht erforderlich, besonders gereinigtes Thionylchlorid zu verwenden.

Im allgemeinen erfolgt die Umsetzung mit Thionylchlorid bei Temperaturen von 30 bis 150 °C, vorzugsweise von 50 bis 130 °C und insbesondere bevorzugt von 60 bis 120 °C.

Es ist im allgemeinen vorteilhaft, durch Reglung der Zutropfgeschwindigkeit des Thionylchlorids die Reaktionsgeschwindigkeit zu steuern.

Die Umsetzung mit Thionylchlorid kann bevorzugt bei Normaldruck, jedoch auch bei vermindertem oder erhöhtem Druck bis zu 10 Bar, insbesondere bis etwa 5 Bar, durchgeführt werden.

Es kann vorteilhaft sein, die bei dem erfindungsgemäßen Verfahren entstehenden Abgase beispielsweise in der Blasensäure mit Chlorsulfonsäure zu waschen. Die Temperatur der Chlorsulfonsäure wird dabei im allgemeinen im Bereich von 0 bis 80 °C, insbesondere von 30 bis 70 °C, gehalten.

Bei Rückführung der Wasch-Chlorsulfonsäure in die nächste Reaktionsfolge ergeben sich gegebenenfalls höhere Durchschnittsausbeuten, da gegebenenfalls mit den Abgasen mitgerissene und dann durch Chlorsulfonsäure ausgewaschene Anteile der aromatischen Verbindungen nicht verlorengehen. Vorteilhafterweise erhält man so ein Abgas, aus dem die aromatischen Verbindungen abgetrennt sind, und das daher leichter aufbereitet werden kann.

Nach Beendigung der Reaktion, was an der Beendigung der Gasentwicklung oder mit bekannten analytischen Methoden festgestellt werden kann, wird das überschüssige Thionylchlorid, gegebenenfalls das Lösungsmittel und eventuell nicht umgesetzte aromatische Verbindung oder nicht umgesetzte Chlorsulfonsäure abgetrennt, beispielsweise vorzugsweise durch Destillation. Das verbleibende aromatische Sulfonsäurechlorid kann, falls erforderlich, beispielsweise durch Destillation oder Kristallisation gereinigt werden.'

In einer bevorzugten Herstellung beispielsweise des Benzolsulfonsäurechlorids oder des Diphenylätherdisulfochlorids kann man bezüglich der Stufe 1

a) den Aromaten gegebenenfalls zusammen mit den Sulfonierungshilfsmitteln, letztere vollständig oder teilweise, vorlegen und die Chlorsulfonsäure eventuell mit der Restmenge Hilfsstoffe nach Maßgabe der Reaktionsgeschwindigkeit zugeben oder

b) in einer bevorzugten Arbeitsweise die Chlorsulfonsäure und gegebenenfalls die Sulfonierungshilfsstoffe jeweils ganz oder teilweise vorlegen und den Aromaten, gegebenenfalls synchron mit der restlichen Chlorsulfonsäure und den Hilfsstoffen, nach Maßgabe der Reaktionsgeschwindigkeit zugeben.

Bei der Herstellung von Benzolsulfonsäurechlorid wird die Stufevorzugsweise bei 20-80 °C, insbesondere bei 40-70 °C durchgeführt.

Überraschenderweise findet man für die Gesamtreaktion (Stufe 1 und Stufe 2) eine Abnahme der Sulfonbildung unter gleichzeitiger Ausbeutesteigerung an Benzolsulfonsäurechlorid, wenn in Stufe 1 in Gegenwart von Sulfonierungshilfsstoffen bei erhöhter Temperatur umgesetzt wird. In Abwesenhiet der Sulfonierungshilfsstoffe sinkt dagegen bei erhöhter Temperatur die Ausbeute an Benzolsulfonsäurechlorid ab.

Das erfindungsgemäße Verfahren kann bezüglich der einzelnen Stufen sowohol diskontinuierlich als auch kontinuierlich durchgeführt werden. Vorteilhafterweise erhält man durch das erfindungsgemäße Verfahren verbesserte Ausbeuten bezogen sowohl auf die aromatische Verbindung als auch insbesondere auf die Chlorsulfonsäure.

Durch das erfindungsgemäße Verfahren entfallen weitgehend der Zwangsanfall an Dünnsäure und

die damit verbundenen ökologischen Probleme. Man destilliert überschüssiges Thionylchlorid, gegebenenfalls Lösungsmittel und gegebenenfalls nicht umgesetzte Chlorsulfonsäure oder nicht umgesetzte aromatische Verbindungen, deren Rückführung möglich ist, ab und erhält anschließend aus dem Rückstand das gebildete aromatische Sulfonsäurechlorid.

Beispiels 1 bis 14

Benzolsulfonsäurechlorid

Ein Kolben wird mit Rührer, Tropftrichter, Innenthermometer und Rückflußkühler mit Abgasableitung, gegebenenfalls über eine Gaswaschflasche, versehen.

Man legt die Chlorsulfonsäure und gegebenenfalls das jeweilige Sulfonierungshilfsmittel vor und tropft bei der in Tabelle I angegebenen Temperatur innerhalb von 4 Stunden langsam Benzol ein. Unter Halten der Temperatur wird bis zum Ende der Gasentwicklung nachgerührt.

Anschließend wird die Reaktionsmischung bei 70-75 °C allmählich mit Thionylchlorid versetzt und bei dieser Temperatur bis zum Ende der erneuten Gasentwicklung gerührt.

Das überschüssige Thionylchlorid und eventuell nicht umgesetztes Benzol werden in einem Rotationsverdampfer bei etwa 130 mbar abdestilliert.

Der verbleibende Rückstand wird bei 7-13 mbar über eine Destillationsbrücke destilliert. Tabelle I zeigt die jeweils erhaltenen Ausbeuten. Nach GC-Analysen ist der Gehalt stets besser als 99 Prozent.

In den Beispielen 1 bis 3 und 12 werden die gesamten Abgase der Reaktion mit Chlorsulfonsäure von 30 °C gewaschen. Diese Wasch-Chlorsulfonsäure wird dann in einer in gleicher Weise durchgeführten zweiten Reaktionsfolge anteilig mit eingesetzt, die herbei resultierende Ausbeute ist aus Tabelle I ersichtlich.

Die Destillationsrückstände werden mit warmem Alkali behandelt, der unlösliche Anteil ist Diphenylsulfon.

(Siehe Tabelle Seite 6 f.)

| Beispiel Nr. | $ClSO_2H$ (Mol) | Benzol (Mol) | $SOCl_2$ (Mol) | Zusatz Art | (Mol) | Sulfonier Temp. °C | Sulfochlorid % d. Th. * | Sulfon % d. Th. |
|---|---|---|---|---|---|---|---|---|
| 1 | 3.15 ** | 3 | 6 | DMF | 0.15 | 70 | 91.5 *** | 5.0 |
| 2 | 3.15 ** | 3 | 6 | DMF | 0.09 | 70 | 90.7 | 6.6 |
| 3 | 1.05 ** | 1 | 2 | DMF | 0.05 | 100 | 84.9 | 8.5 |
| 4 | 1.05 | 1 | 2 | DMF | 0.05 | 70 | 89.8 | 4.3 |
| 5 | 1 | 1 | 2 | DMF | 0.05 | 70 | 87.5 | 4.5 |
| 6 | 1 | 1 | 2 | $CH_2COOH$ | 0.05 | 70 | 85.8 | 5.2 |
| 7 | 1 | 1 | 2 | $H_3PO_4$ | 0.05 | 80 | 85.2 | 6.9 |
| 8 | 1 | 1 | 2 | DMF | 0.05 | 20 | 84.0 | 9.2 |
| 9 | 1 | 1 | 2 | DMF | 0.10 | 70 | 80.4 | 3.4 |
| 10 | 1 | 1 | 2 | DMF | 0.02 | 70 | 84.0 | 7.1 |
| 11 | 1 | 1 | 2 | DMF | 0.01 | 70 | 76.0 | 8.1 |
| 12 (Vergleich) | 1.05 ** | 1 | 1 | — | — | 20 | 83.5 | 14.7 |
| 13 (Vergleich) | 1 | 1 | 2 | — | — | 20 | 79.1 | 14.4 |
| 14 (Vergleich) | 1 | 1 | 2 | — | — | 70 | 73.7 | 17.4 |

* bezogen auf eingesetztes Benzol

** unter Mitverwendung von « Wasch-Chlorsulfonsaure (siehe exp. Beschreibung)

*** Durchschnittsergebnis aus 5 Versuchen, Schwankungsbreite 90.7 bis 94.3 % d. Th.

Beispiele 15 bis 25

Nach der in den Beispielen 1 bis 14 beschriebenen Arbeitsweise werden die aus Tabelle II ersichtlichen Aromaten in einer standardisierten Arbeitsweise entsprechend Beispiel 5 umgesetzt.

Die Temperatur in Stufe 1 (Umsetzung mit Chlorsulfonsäure) und Stufe 2 (Umsetzung mit Thionylchlorid) beträgt jeweils 70 °C. Es werden jeweils 5 Mol-% Dimethylformamid als Sulfonierungshilfsmittel eingesetzt.

Die Beispiele 15 bis 25 sind, da standardisiert verfahren wurde, bezüglich der Temperaturführung nicht optimiert.

| Beispiel Nr. | Aromatische Verbindung [q] | [Mol] | $ClSO_3H$ [q] | $ClSO_3H$ [Mol] | $SOCl_2$ [q] | $SOCl_2$ [Mol] | Aromatisches Sulfonsäurechlorid [q] | Reinheit * | % d. Th. |
|---|---|---|---|---|---|---|---|---|---|
| 15 | Toluol | | | | | | Toluolsulfonsäurechlorid | | |
| | 92 | 1 | | 1 | | 2 | 153 | 30.0 % o-<br>70.0 % p- | 80 |
| 16 | Cumol | | | | | | 4-Isopropyl-benzolsulfonsäurechlorid | | |
| | 120 | 1 | | 1 | | 2 | 193 | 94.4 | 83 |
| 17 | Tetralin | | | | | | 1,2,3,4-Tetrahydronaphthalin-6-sulfonsäurechlorid | | |
| | 132 | 1 | | 1 | | 2 | 195 | 86.5 %<br>11.1 % Isomeres | 71<br>9 |
| 18 | p-Xylol | | | | | | 2,5-Dimethyl-benzolsulfonsäurechlorid | | |
| | 106 | 1 | | 1 | | 2 | 177 | 99.0 | 86 |
| 19 | Diphenylmethan | | | | | | Diphenylmethan-4,4'-disulfonsäurechlorid ** | | |
| | 168 | 1 | | 2 | | 4 | 224 | Fp. 121 °C | 61 |
| 20 | Diphenyl | | | | | | 4,4'-Diphenyldisulfonsäurechlorid ** | | |
| | 154 | 1 | | 2 | | 4 | 218 | Fp. 200 °C | 62 |
| 21 | o-Chlor-toluol | | | | | | 2-Chlor-toluol-5-sulfonsäurechlorid | | |
| | 127 | 1 | | 1 | | 2 | 187 g | 78.6<br>16.1 Isomeres | 65<br>13 |
| 22 | 2,6-Dichlor-toluol | | | | | | 2,4-Dichlor-3-methyl-benzolsulfonsäurechlorid | | |
| | 161 | 1 | | 1 | | 2 | 179 | 91.0 | 63 |
| 23 | Chlorbenzol | | | | | | p-Chlor-benzolsulfonsäurechlorid | | |
| | 113 | 1 | | 1 | | 2 | 189 | 99.3 | 89 |

(Fortsetzung)

| Beispiel Nr. | Aromatische Verbindung [g] | [Mol] | $ClSO_3H$ [g] | [Mol] | $SOCl_2$ [g] | [Mol] | Aromatisches Sulfonsäurechlorid [g] | Reinheit * | % d. Th. |
|---|---|---|---|---|---|---|---|---|---|
| 24 | Fluorbenzol 96 | 1 | | 1 | | 2 | p-Fluor-benzolsulfonsäurechlorid 164 | 96.0 | 81 |
| 25 | o-Dichlorbenzol 147 | 1 | | 1 | | 1 | 3,4-Dichlor-benzolsulfonsäurechlorid 200 | 98.5 | 80 |

* entsprechend gaschromatographischer Analyse

** wegen des hohen Siedepunktes statt Destillation Umkristallisation

## Beispiel 26

Zu 5 g Chlorsulfonsäure in 120 $cm^3$ Äthylenchlorid tropft man innerhalb einer Stunde bei 50° gleichzeitig 114 g Chlorsulfonsäure (66 $cm^3$) und 85 g Diphenyläther, gelöst in 50 $cm^3$ Äthylenchlorid (132 $cm^3$). Man rührt das Gemisch 30 Min. bei 50° nach, läßt bei dieser Temperatur innerhalb 90 Min. 300 g Thionylchlorid (2,52 Mol = 1,52 Mol Überschuß) zufließen und erhitzt das Gemisch 6 Std. am Rückfluß bei 80-85°. Überschüssiges Thionylchlorid und Äthylenchlorid werden bis zu einer Sumpf temperatur von 130-140°, zuletzt bei 13 mbar, abdestilliert. Das flüssig auf ein Blech ausgetragene Produkt wird nach dem Erstarren und Zerreiben bei 40° i. Vak. getrocknet. Ausbeute : 178 g = 97 % d. Th., Fp. 123,5-128°. DC : 0,1 % Monosulfochlorid,
2,0 % unbekannte Nebenprodukte.

**Patentansprüche**

1. Verfahren zur Herstellung von Sulfonsäurechloriden der Formel

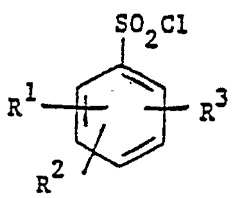

(I)

worin $R^1$, $R^2$ und $R^3$ gleich oder verschieden sind und Wasserstoff, einen niederen Alkyl- oder einen Cycloalkylrest, Halogen, Aryl, Aralkyl, Aryläther oder einen Rest $—SO_2Cl$, $—SO_2Aryl$,

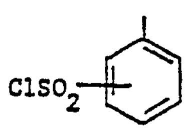, 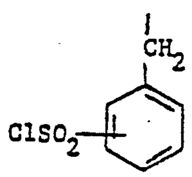, 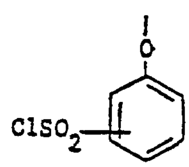 oder 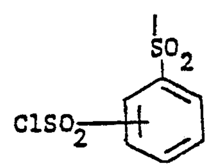

bedeuten
oder wo benachbarte Reste $R^1$ und $R^2$ zu einem gegebenenfalls durch eine Sulfonsäurechloridgruppe substituierten cycloaliphatischen, aromatischen oder heteroaromatischen Ring verknüpft sind,
wobei die Reste $R^1$ bis $R^3$ durch weitere Reste wie Halogen, Niederalkyl, Aryl, Aroxy, Alkoxy und Aralkyl substituiert sein können, durch Umsetzung einer aromatischen Verbindung der Formel

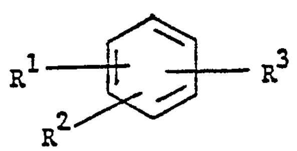

(II)

worin $R^1$, $R^2$ und $R^3$ die oben genannte Bedeutung haben, mit Chlorsulfonsäure und Thionylchlorid, dadurch gekennzeichnet, daß man zuerst die aromatische Verbindung und Chlorsulfonsäure in stöchiometrischer Menge bis zu einem Überschuß von 20 Mol-%, bezogen auf jede einzuführende Sulfonsäurechlorid-Gruppe, in Gegenwart eines Sulfonierungshilfsmittels und gegebenenfalls in Gegenwart eines Lösungsmittels zur Reaktion bringt und dann mit überschüssigem Thionylchlorid umsetzt.

2. Verfahren nach Anspruch 1, dadurch gekennzeichnet, daß die Chlorsulfonsäure vorgelegt und die aromatische Verbindung nach Maßgabe der Reaktionsgeschwindigkeit zugegeben wird.

3. Verfahren nach Anspruch 1 bis 2, dadurch gekennzeichnet, daß die aromatischen Verbindungen und Chlorsulfonsäure im stöchiometrischen Verhältnis eingesetzt werden.

4. Verfahren nach Anspruch 1 bis 3, dadurch gekennzeichnet, daß das in der ersten Stufe erhaltene Reaktionsgemisch in der zweiten Stufe mit Thionylchlorid einer Menge von bis zu 5 Äquivalenten, bezogen auf jede einzuführende Sulfonsäurechlorid-Gruppe, umgesetzt wird.

5. Verfahren nach Anspruch 1 bis 4, dadurch gekennzeichnet, daß in kontinuierlicher Arbeitsweise die Abgase mit Chlorsulfonsäure gewaschen werden und die so erhaltene Chlorsulfonsäure in das Verfahren zurückgeführt wird.

**Claims**

1. Process for the preparation of sulphonic acid chlorides of the formula

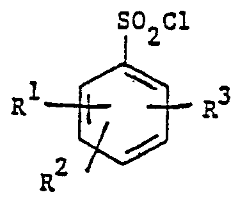

(I)

wherein $R^1$, $R^2$ and $R^3$ are identical or different and denote hydrogen, a lower alkyl or a cycloalkyl radical, halogen, aryl, aralkyl, aryl ether or a radical $—SO_2Cl$, $—SO_2aryl$,

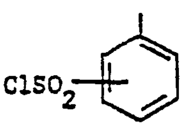, 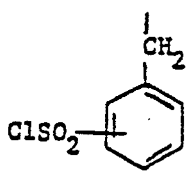, 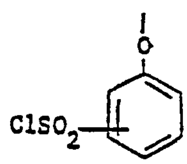 or 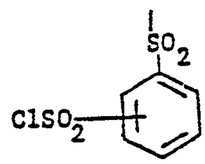

or wherein adjacent radicals $R^1$ and $R^2$ are linked to form a cycloaliphatic, aromatic or heteroaromatic ring which is optionally substituted by a sulphonic acid chloride group, it being possible for the radicals $R^1$ to $R^3$ to be substituted by further radicals such as halogen, lower alkyl, aryl, aroxy, alkoxy and aralkyl, by reacting an aromatic compound of the formula

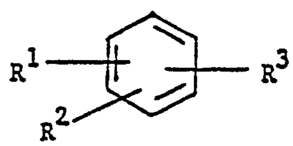

(II)

wherein $R^1$, $R^2$ and $R^3$ have the abovementioned meaning, with chlorosulphonic acid and thionyl chloride, characterised in that the aromatic compound and chlorosulphonic acid in a stoichiometric amount up to an excess of 20 mol %, based on each sulphonic acid group to be introduced, are first reacted, in the presence of a sulphonation auxiliary and optionally in the presence of a solvent and the product is then reacted with excess thionyl chloride.

2. Process according to Claim 1, characterised in that the chlorosulphonic acid is initially introduced and the aromatic compound is added at a rate depending on the rate of reaction.

3. Process according to Claim 1 to 2, characterised in that the aromatic compounds and chlorosulphonic acid are used in a stoichiometric ratio.

4. Process according to Claim 1 to 3, characterised in that the reaction mixture obtained in the first stage is reacted in the second stage with thionyl chloride in a quantity of up to 5 equivalents, based on each sulphonic acid chloride group to be introduced.

5. Process according to claim 1 to 4, characterised in that the off-gases are washed with chlorosulphonic acid in a continuous procedure and the chlorosulphonic acid thus obtained is recycled to the process.

**Revendications**

1. Procédé pour la préparation de chlorure d'acide sulfonique de formule

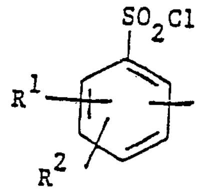

(I)

dans laquelle $R^1$, $R^2$ et $R^3$ sont identiques ou différents et représentent l'hydrogène, un reste alkyle inférieur ou cycloalkyle, un halogène, un groupe aryle, un groupe arylalkyle, aryléther ou un reste $—SO_2Cl$, $—SO_2$ aryle,

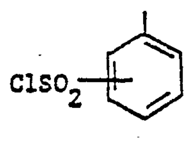, 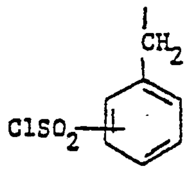, 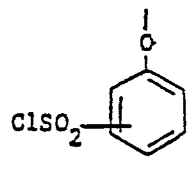 ou 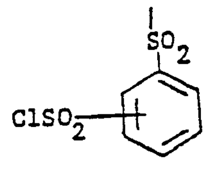

ou bien les restes voisins $R^1$ et $R^2$ sont reliés pour former un noyau cycloaliphatique, aromatique ou hétéroaromatique éventuellement substitué par un groupe chlorure d'acide sulfonique, les restes $R^1$ à $R^3$ pouvant être substitués par d'autres restes tels qu'halogène, alkyle inférieur, aryle, aroxy, alcoxy et arylalkyle, par réaction d'un composé aromatique de formule

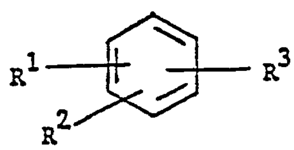

(II)

dans laquelle $R^1$, $R^2$ et $R^3$ ont la signification mentionnée ci-dessus, avec l'acide chlorosulfonique et le chlorure de thionyle, caractérisé en ce que l'on fait d'abord réagir le composé aromatique et l'acide chlorosulfonique en quantié stoechiométrique jusqu'à un excès de 20 moles % par rapport à chaque groupe chlorure d'acide sulfonique à introduire en présence d'un agent auxiliaire de sulfonation et éventuellement en présence d'un solvant, et on fait ensuite réagir avec le chlorure de thionyle en excès.

2. Procédé selon la revendication 1, caractérisé en ce que l'on charge au préalable l'acide chlorosulfonique et on ajoute le composé aromatique dans la mesure de la vitesse de réaction.

3. Procédé selon la revendication 1 à 2, caractérisé en ce que l'on utilise les composés aromatiques et l'acide chlorosulfonique dans le rapport stoechiométrique.

4. Procédé selon la revendication 1 à 3, caractérisé en ce que l'on fait réagir dans la seconde étape le mélange de réaction obtenu dans la première étape avec le chlorure de thionyle en quantité allant jusqu'à 5 équivalents, par rapport à chaque groupe chlorure d'acide sulfonique à introduire.

5. Procédé selon la revendication 1 à 4, caractérisé en ce que, dans le mode opératoire continu, on lave le gaz résiduaire par l'acide chlorosulfonique et on recycle dans le procédé l'acide chlorosulfonique ainsi obtenu.